# EUROPEAN PATENT APPLICATION

(11) **EP 4 407 308 A1**
(43) Date of publication of application: **31.07.2024**
(21) Application number: 22872830.9
(22) Date of filing: 16.09.2022
(51) Int. Cl.: G01N 27/416, G01N 27/04, G01N 27/26, G01N 27/30

(54) **METHOD FOR SENSING AND DEVICE FOR MEASURING VOLATILE FATTY ACID**

(30) Priority: 21.09.2021 JP 2021153071
(71) Applicant: National Institute for Materials Science, Tsukuba-shi, Ibaraki 305-0047 (JP)
(72) Inventor: KAWAKITA Jin, Tsukuba-shi, Ibaraki 305-0047 (JP); ICHINOSE Izumi, Tsukuba-shi, Ibaraki 305-0047 (JP)
(74) Representative: Dehns
(86) International application number: PCT/JP2022/034697
(87) International publication number: WO 2023/048076

(57) **Abstract**

The present invention provides a method for sensing volatile fatty acid with high sensitivity and measuring volatile fatty acid with high accuracy and provides a device for such sensing and measurement. The method for sensing volatile fatty acid of the present invention includes placing a sample in an enclosed space together with a resistance measurement device, wherein the sample is allowed to contain volatile fatty acid, the resistance measurement device measures an electrical characteristic caused by electrical resistance between at least two electrodes disposed to be adjacent to each other at a small spacing on an insulating substrate, and water vapor exists in the enclosed space, measuring the electrical characteristic when a space near the at least two electrodes has a relative humidity satisfying a humidity condition of immediately before occurrence of dew condensation between the at least two electrodes, and comparing an obtained measurement result with reference data obtained under a predetermined condition.

## Description

### Technical Field

The present invention relates to a method for sensing volatile fatty acid and a device for measuring volatile fatty acid.

### Background Art

Volatile fatty acids (VFAs) such as propionic acid, butyric acid, and acetic acid are substances that greatly affect the productivity and quality improvement in dairy farming of cattle and the like. Cattle discharge a large amount of methane gas, which promotes global warming, at the time of digesting food. It has been reported that the amount of methane gas discharged can be significantly reduced by controlling VFAs in cattle rumina (first stomachs).

From such a background, there are a demand for a method for sensing and quantitatively measuring VFA, particularly, VFA volatilized from a liquid part in a cow rumen and a demand for a device for such sensing and measurement. In the present description, concerning VFAs, the term "sensing" means confirming presence or absence of VFA, and the term "measurement" means measuring and determining (quantifying) the amount of VFA.

Conventionally, a method has been attempted in which VFA is adsorbed to an adsorbent, a weight change and an infrared absorption spectrum change of the adsorbent due to adsorption of VFA are monitored, and thus VFA is sensed and measured.

However, this method has a problem that the sensing sensitivity and the measurement accuracy are low and the device is relatively large. Note that a practical sensing sensitivity and a practical measurement accuracy are required such that 25 mM (millimole) of a liquid of a liquid part in a cow rumen can be sensed and measured.

### Citation List

### Patent Literature

Patent Literature 1: WO 2016/13544 A

### Summary of Invention

### Technical Problem

An object of the present invention is to provide a method for sensing VFA with high sensitivity and measuring VFA with high accuracy and to provide a device for such sensing and measurement.

### Solution to Problem

The inventors have found that VFA contained in a sample can be sensed with high sensitivity and quantified with high accuracy by measuring an electrical characteristic caused by electrical resistance between electrodes with a small spacing in a humidity environment of immediately before occurrence of dew condensation, and has conceived the present invention.

Configurations of the present invention will be described below.

### (Configuration 1)

A method for sensing volatile fatty acid, the method comprising:
placing a sample in an enclosed space together with a resistance measurement device, wherein the sample is allowed to contain volatile fatty acid, the resistance measurement device measures an electrical characteristic caused by electrical resistance between at least two electrodes disposed to be adjacent to each other at a small spacing on an insulating substrate, and water vapor exists in the enclosed space;
measuring the electrical characteristic when a space near the at least two electrodes has a relative humidity satisfying a humidity condition of immediately before occurrence of dew condensation between the at least two electrodes; and
comparing an obtained measurement result with reference data obtained under a predetermined condition.

### (Configuration 2)

The method for sensing volatile fatty acid according to Configuration 1, wherein the reference data is obtained by measuring the electrical characteristic using the sample when the space near the at least two electrodes has a relative humidity satisfying a humidity condition that does not cause dew condensation between the at least two electrodes, or obtained by measuring the electrical characteristic using a reference sample not containing volatile fatty acid or a reference sample containing volatile fatty acid at a certain rate when the space near the at least two electrodes has a relative humidity satisfying the humidity condition of immediately before occurrence of dew condensation between the at least two electrodes.

### (Configuration 3)

The method for sensing volatile fatty acid according to Configuration 1 or 2, wherein the relative humidity is 80% or more and less than 100% under the humidity condition of immediately before occurrence of dew condensation between the at least two electrodes.

### (Configuration 4)

The method for sensing volatile fatty acid according to Configuration 3, wherein the relative humidity is 80% or more and 95% or less under the humidity condition of immediately before occurrence of dew condensation between the at least two electrodes.

### (Configuration 5)

The method for sensing volatile fatty acid according to any one of Configurations 1 to 4, wherein the at least two electrodes have a configuration in which a first thin wire electrode and a second thin wire electrode are alternately disposed in juxtaposition with each other in at least a partial region on the insulating substrate.

### (Configuration 6)

The method for sensing volatile fatty acid according to Configuration 5, wherein the first thin wire electrode and the second thin wire electrode are alternately disposed at a specified spacing.

### (Configuration 7)

The method for sensing volatile fatty acid according to Configuration 6, wherein the spacing is 100 nm or more and 1000 nm or less.

### (Configuration 8)

The method for sensing volatile fatty acid according to any one of Configurations 5 to 7, wherein
the first thin wire electrode has an exposed surface including at least a part on which a first metal is formed, the second thin wire electrode has an exposed surface including at least a part on which a second metal is formed, the second metal being different from the first metal, and
a current flowing between the first thin wire electrode and the second thin wire electrode is measured.

### (Configuration 9)

The method for sensing volatile fatty acid according to Configuration 8, wherein the first metal is selected from the group consisting of gold, platinum, silver, titanium, an alloy thereof, and carbon.

### (Configuration 10)

The method for sensing volatile fatty acid according to Configuration 8 or 9, wherein the second metal is selected from the group consisting of silver, copper, iron, zinc, nickel, cobalt, aluminum, tin, chromium, molybdenum, manganese, magnesium, and an alloy thereof.

### (Configuration 11)

The method for sensing volatile fatty acid according to any one of Configurations 1 to 10, wherein the insulating substrate has a hydrophilic surface.

### (Configuration 12)

The method for sensing volatile fatty acid according to any one of Configurations 1 to 11, wherein the relative humidity is controlled by a temperature adjustment device thermally connected to the resistance measurement device.

### (Configuration 13)

The method for sensing volatile fatty acid according to Configuration 12, wherein the temperature adjustment device is a Peltier device.

### (Configuration 14)

The method for sensing volatile fatty acid according to any one of Configurations 1 to 13, wherein the sample is in a form of an aqueous solution.

### (Configuration 15)

A device for measuring volatile fatty acid, the device comprising a resistance measurement device, a humidity measurement means, a data extraction means, and a data analysis means, wherein
the resistance measurement device is configured to include at least two electrodes disposed to be adjacent to each other at a small spacing on an insulating substrate, to measure an electrical characteristic caused by electrical resistance between the at least two electrodes, and to output an obtained result,
the humidity measurement means is arranged to be adjacent to the resistance measurement device, and is configured to measure a relative humidity of a space near the at least two electrodes of the resistance measurement device, and to output an obtained result,
the data extraction means is configured to extract output data from the resistance measurement device when the relative humidity measured by the humidity measurement means satisfies a predetermined humidity condition, and
the data analysis means is configured to compare the output data extracted by the data extraction means with calibration curve data obtained in advance, and to output an obtained result.

### (Configuration 16)

The device for measuring volatile fatty acid according to Configuration 15, wherein the at least two electrodes have a configuration in which a first thin wire electrode and a second thin wire electrode are alternately disposed in juxtaposition with each other in at least a partial region on the insulating substrate.

### (Configuration 17)

The device for measuring volatile fatty acid according to Configuration 16, wherein the first thin wire electrode and the second thin wire electrode are alternately disposed at a specified spacing.

### (Configuration 18)

The device for measuring volatile fatty acid according to Configuration 17, wherein the spacing is 100 nm or more and 1000 nm or less.

### (Configuration 19)

The device for measuring volatile fatty acid according to any one of Configurations 16 to 18, wherein
the first thin wire electrode has an exposed surface including at least a part on which a first metal is formed, the second thin wire electrode has an exposed surface including at least a part on which a second metal is formed, the second metal being different from the first metal, and
the resistance measurement device is configured to measure a current flowing between the first thin wire electrode and the second thin wire electrode, and to output an obtained result.

### (Configuration 20)

The device for measuring volatile fatty acid according to any one of Configurations 16 to 19, wherein the humidity measurement means includes humidity measurement electrodes that determine a humidity based on electrical resistance between the humidity measurement electrodes, and a material used for constituting the first thin wire electrode or the second thin wire electrode is used for constituting the humidity measurement electrodes.

### Advantageous Effects of Invention

According to the present invention, it is possible to provide a method for sensing VFA with high sensitivity and measuring VFA with high accuracy and to provide a device for such sensing and measurement.

### Brief Description of Drawings

Fig. 1 is a schematic configuration diagram explaining a configuration of a VFA sensing and measurement device of the present invention.
Fig. 2 shows structural drawings explaining a configuration of a resistance measurement device (galvanic-type sensor). Fig. 2(a) is a plan view and Fig. 2(b) is a sectional view.
Fig. 3 is an explanatory view explaining an operation principle of a resistance measurement device that is a galvanic-type sensor.
Fig. 4 is an explanatory view for explaining a method for sensing and measuring VFA of the present invention.
Fig. 5 is an explanatory graph for explaining a method of the present invention on the basis of temporal changes in sensor output and platinum resistance obtained using a prototype VFA sensing and measurement system.
Fig. 6 is a characteristic graph indicating the propionic acid concentration dependence of the sensor output shown in Fig. 5.
Fig. 7 is a characteristic graph indicating the propionic acid concentration dependence of the sensor output shown in Fig. 5.

### Description of Embodiments

### <Concept>

In the method for sensing and measuring VFA of the present invention, a sample is placed in an enclosed space together with a resistance measurement device, wherein the sample is allowed to contain volatile fatty acid, and water vapor exists in the enclosed space. An output from the resistance measurement device is monitored when a space near electrodes provided in the resistance measurement device has a relative humidity satisfying a humidity condition of immediately before occurrence of dew condensation between the electrodes.

The resistance measurement device is configured to measure an electrical characteristic (electrical resistance, conductivity, or current value) caused by electrical resistance between at least two electrodes disposed to be adjacent to each other at a small spacing on an insulating substrate, and an output from the resistance measurement device is a measurement result of the electrical characteristic.

Specific examples of such a resistance measurement device can include a galvanic-type sensor that has two or more electrodes exposed so as to be in contact with the outside air (intended to be a water vapor atmosphere in the enclosed space in the present invention) on an insulating substrate. In the galvanic-type sensor, at least two different kinds of metals are used as the electrode materials, and a galvanic current flowing between the electrodes is measured. Such a galvanic-type sensor is disclosed in, for example, Patent Literature 1.

Specific examples of the resistance measurement device can further include a resistance measurement sensor that has two or more electrodes exposed so as to be in contact with the outside air on an insulating substrate. In the resistance measurement sensor, the electrodes are made of the same material. A voltage is applied between the electrodes, and a current flowing between the electrodes is measured, or a bridge circuit is provided and a resistance value is measured from the circuit.

Among them, the galvanic-type sensor is small and not necessarily requiring an external power supply, and can be particularly preferably used. Details of an aspect in which the galvanic-type sensor is used as the resistance measurement device in the method for sensing and measuring VFA of the present invention will be described below.

The inventors have found that, in a state where VFA exists in a water vapor atmosphere, if an electrical characteristic caused by electrical resistance between the electrodes of the resistance measurement device is measured when the space near the electrodes has a relative humidity satisfying a humidity condition of immediately before occurrence of dew condensation between the electrodes, an output from the resistance measurement device depends on the concentration of VFA in the atmosphere, and VFA can be sensed with high sensitivity and quantified with high accuracy.

Meanwhile, when an electrical characteristic caused by electrical resistance between the electrodes was measured in a state where dew condensation occurred between the electrodes of the resistance measurement device, the output from the resistance measurement device was not stable until the spacing between the electrodes was completely filled with a liquid containing water and VFA, and the output did not correlate with the concentration of VFA in the atmosphere. Furthermore, there was a problem that the sensing sensitivity and the measurement accuracy for VFA were not high even in a state where the spacing between the electrodes of the resistance measurement device was completely filled with the liquid containing water and VFA.

Here, as the humidity condition of immediately before occurrence of dew condensation between the electrodes of the resistance measurement device, the relative humidity of the space near the electrodes of the resistance measurement device is preferably 80% or more and less than 100%, and more preferably 80% or more and 95% or less from the viewpoint of sensing sensitivity and measurement accuracy for VFA through many experiments. The space near the electrodes of the resistance measurement device means a space including at least the outside air in contact with the electrodes of the resistance measurement device in the enclosed space in which the device is placed, and typically, a space including an atmosphere around the device is intended. Specifically, for example, the relative humidity of the space near the electrodes of the resistance measurement device can be measured by arranging any humidity measurement means to be adjacent to the resistance measurement device in the enclosed space.

The sensing and the measurement of VFA according to the present invention are considered to be based on the following mechanism.

If the measurement is performed when the space near the electrodes of the resistance measurement device has a relative humidity satisfying the humidity condition of immediately before occurrence of dew condensation between the electrodes, water molecules derived from water vapor and VFA volatilized from the sample and floating in the atmosphere in the enclosed space are co-adsorbed to a place where a target electrical characteristic is measured by the resistance measurement device, that is, onto an exposed surface of the insulating substrate between the electrodes of the resistance measurement device. Then, protons (H⁺) generated by ionization become carriers, and the carriers move in a hopping manner via the water molecules or the adsorbed VFA to cause a change in the electrical resistance (that may also be referred to as conductivity) between the electrodes. In other words, it can be said that under the humidity condition of immediately before occurrence of dew condensation between the electrodes of the resistance measurement device, a condensation phenomenon of water vapor in the atmosphere does not occur on an exposed surface of the insulating substrate between the electrodes, but the exposed surface is in a state that can cause many (frequent) adsorption phenomena of water molecules derived from water vapor. Such a state is usually considered to occur in a certain time range (time interval) rather than at a certain time point (for an instant). In the present invention, this phenomenon is used to compare the measurement result obtained under the above condition with data obtained by measuring the electrical resistance between the electrodes using the same sample when the relative humidity is in a humidity condition that does not cause dew condensation between the electrodes, and thus VFA in the sample can be sensed. Alternatively, VFA in the sample can also be sensed by comparing the measurement result obtained under the above condition with data obtained by measuring the electrical resistance between the electrodes using a reference sample not containing VFA or a reference sample containing VFA at a certain rate under the above condition (that is, when the relative humidity satisfies the humidity condition of immediately before occurrence of dew condensation between the electrodes). Furthermore, the amount of the above-described protons largely and monotonically depends on the amount of VFA, and therefore the amount of VFA in the sample can be determined (quantified) by using a calibration curve. Moreover, in this mechanism, a kind of interface phenomenon is used, and therefore high sensing sensitivity and high measurement accuracy can be easily obtained while the device has a compact configuration. For adsorbing more water molecules on an exposed surface of the insulating substrate between the electrodes, the insulating substrate preferably has a hydrophilic surface. If an insulating substrate having a hydrophilic surface is used, co-adsorption of water molecules and VFA is promoted, and thus the sensing sensitivity and the measurement accuracy of VFA are enhanced, and in addition, the stability of output from the resistance measurement device and the reproducibility of output data are also improved. Here, the state that the surface is hydrophilic means that the surface has a contact angle with water of 0° or more and 50° or less.

Meanwhile, if the electrical resistance between the electrodes is measured under a humidity condition that causes dew condensation between the electrodes of the resistance measurement device, droplets *per se* are formed between the electrodes. However, the formation process of such droplets affects an electrical characteristic (electrical resistance), and thus the electrical characteristic behaves in accordance with the probability theory. As a result, the reproducibility of output data from the resistance measurement device cannot be obtained, VFA contained in the sample is difficult to sense accurately, and the amount of VFA cannot be grasped.

A method is also conceivable in which a layer of water is formed in advance on an exposed surface of an insulating substrate between electrodes, VFA volatilized from a sample is dissolved in the layer, and the conductivity between the electrodes is measured. However, the solubility of VFA in water is not high, and therefore this method has problems of difficulty in obtaining high sensing sensitivity and high measurement accuracy and a large size of the device.

### < VFA Sensing and Measurement Device >

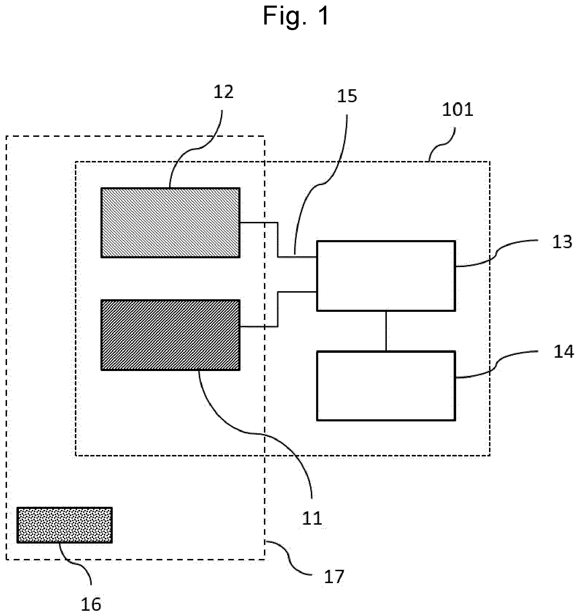

Fig. 1 shows a configuration of the VFA sensing and measurement device of the present invention.

The VFA sensing and measurement device 101 of the present invention includes a resistance measurement device 11, a humidity measurement means 12, a data extraction means 13, and a data analysis means 14.

Output data from the resistance measurement device 11 and the humidity measurement means 12 is sent to the data extraction means 13 via signal lines 15. The output data from the resistance measurement device 11 extracted by the data extraction means 13 when a predetermined humidity condition is satisfied is sent to the data analysis means 14 via another signal line, and in the data analysis means 14, the data is compared with calibration curve data or the like obtained in advance, and presence or absence of VFA in a sample 16 is confirmed and/or VFA is quantified. The resistance measurement device 11 and the humidity measurement means 12 are arranged in an enclosed space 17 together with the sample 16, wherein water vapor exists in the enclosed space 17.

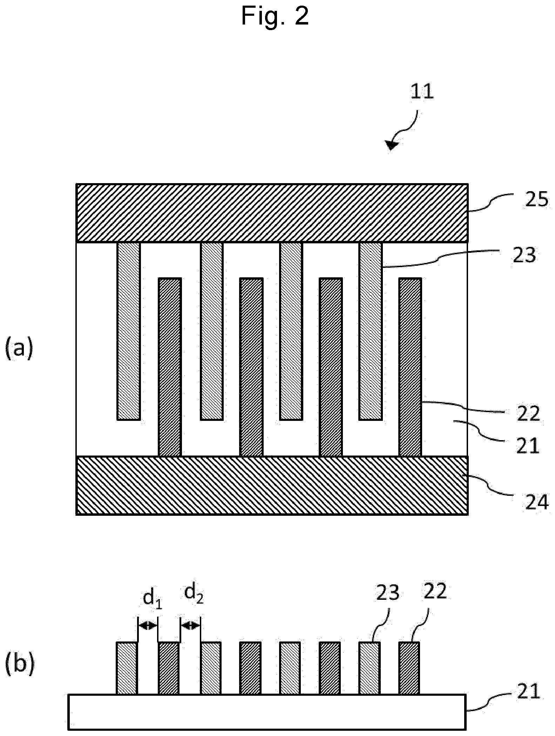

As shown in the plan view of Fig. 2(a), the resistance measurement device 11 includes a substrate (insulating substrate) 21, first thin wire electrodes 22, second thin wire electrodes 23, a first electrode (first collector electrode) 24, and a second electrode (second collector electrode) 25. A first thin wire electrode 22 and a second thin wire electrode 23 are disposed to be adjacent to each other at a small spacing on the insulating substrate 21. The resistance measurement device 11 measures an electrical characteristic caused by electrical resistance between the first thin wire electrodes 22 and the second thin wire electrodes 23.

The first thin wire electrodes 22 and the second thin wire electrodes 23 preferably have a configuration in which the first thin wire electrodes 22 and the second thin wire electrodes 23 are alternately disposed in juxtaposition with each other in at least a partial region on the insulating substrate 21. Such a configuration widens the opposing surfaces of a first thin wire electrode 22 and a second thin wire electrode 23, resulting in an improvement in the sensitivity for VFA and an improvement in the stability (reproducibility) of the obtained result.

Furthermore, the first thin wire electrodes 22 and the second thin wire electrodes 23 are preferably alternately disposed at a specified spacing, that is, it is preferable that the values of d₁ and d₂ in the sectional view of Fig. 2(b) are equal and do not vary in the plane (are constant in the plane). If this spacing is constant, the above-described VFA sensing and measurement mechanism according to the present invention functions effectively to cause a rapid change in the electrical resistance between the first thin wire electrodes 22 and the second thin wire electrodes 23, resulting in an improvement in the sensing sensitivity and an improvement in the measurement accuracy.

The resistance measurement device 11 may be a resistance measurement sensor in which the same conductive material is used for constituting a first thin wire electrode 22 and a second thin wire electrode 23, a voltage is applied between the electrodes via a first electrode 24 and a second electrode 25, a current flowing between the first electrode 24 and the second electrode 25 is measured, and thus the electrical resistance is measured. Alternatively, the resistance measurement device 11 may be a galvanic-type sensor in which different metals are used for constituting a first thin wire electrode 22 and a second thin wire electrode 23, a galvanic current flowing between a first electrode 24 and a second electrode 25 is measured, and thus the electrical resistance is measured. The first thin wire electrode 22 and the first electrode 24 may be constituted of the same conductive material or different conductive materials. The same applies to the second thin wire electrode 23 and the second electrode 25.

Hereinafter, the resistance measurement device 11 will be described in more detail with reference to an example in which the resistance measurement device 11 is a galvanic-type sensor.

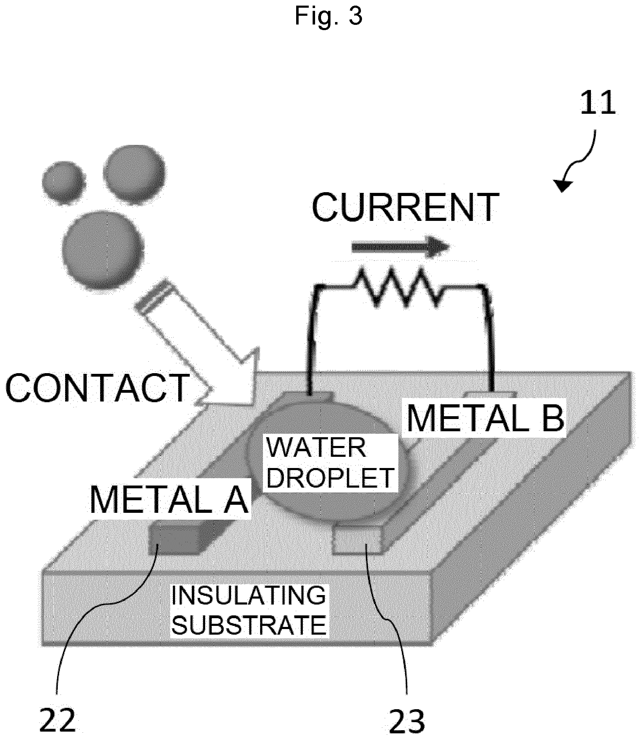

As shown in Fig. 3, a galvanic-type sensor 11 is a current sensing sensor that includes a first thin wire electrode 22 and a second thin wire electrode 23 constituted with different metals (a metal A and a metal B), respectively, and alternately disposed in juxtaposition with each other on an insulating substrate. The galvanic-type sensor 11 utilizes a phenomenon such that when a conductive droplet such as a water droplet contacts both the electrodes of the thin wire electrode pair, a galvanic current flows between the electrodes.

As shown in Figs. 2(a) and 2(b), in the galvanic-type sensor 11, the first thin wire electrode 22 constituted of a first metal and the second thin wire electrode 23 constituted of a second metal or a semiconductor are alternately disposed in juxtaposition with each other on an insulating substrate 21, wherein the second metal is different from the first metal. In other words, the first thin wire electrode 22 and the second thin wire electrode 23 are disposed to be adjacent to each other at a small spacing on the insulating substrate 21. The spacing between the first thin wire electrode 22 and the second thin wire electrode 23 (that is, values of d₁ and d₂ in Fig. 2(b)) is preferably 100 nm or more and 1000 nm or less from the viewpoint of sensing VFA with high sensitivity and measuring VFA with high accuracy.

As the insulating substrate 21, a silicon substrate or the like having a surface on which an oxide film (SiO₂ film) is formed can be preferably used, but the insulating substrate 21 is not limited to a silicon substrate, and various insulating materials can also be used that include plastic such as polycarbonate, rubber, and the like. Examples of the "insulating substrate" in the present application also include substrates having a form in which although the substrate body is a conductor such as a metal, an insulating property with respect to the first thin wire electrode 22 and the second thin wire electrode 23 is obtained by forming an insulating coating or a film coating on the substrate body.

The first thin wire electrode 22 is connected to a first electrode 24, the second thin wire electrode 23 is connected to a second electrode 25, and an electric signal is transmitted to a data extraction means 13 via electrical wirings (not shown) connected to the first electrode 24 and the second electrode 25. Here, an amplifier may be connected to the first electrode 24 and the second electrode 25 to amplify a galvanic current and transmit the electric signal to the data extraction means 13.

The spacing between the first thin wire electrode 22 and the second thin wire electrode 23 may be unoccupied, or may be filled with an insulator. Here, in a case where the spacing is filled with an insulator, the insulator preferably has a hydrophilic surface so that water molecules are easily adsorbed to the surface.

As materials of the first thin wire electrode 22 and the second thin wire electrode 23, the first metal and the second metal having a different electrochemical potential from the first metal are used, respectively, and when water molecules and VFA reach a co-adsorbed state of a certain density or more between the metals, protons (H⁺) generated by ionization become carriers, the carriers move in a hopping manner via the water molecules and the adsorbed VFA to cause a galvanic action between the electrodes, and thus a galvanic current flows.

In a case where a length of the portions, in which the first thin wire electrode 22 and the second thin wire electrode 23 faces each other with approaching each other, is increased, it is possible to increase in the capacity of the cell, and therefore such an arrangement is effective in increasing the galvanic current. Therefore, these thin wire electrodes are preferably arranged substantially in parallel with and approached each other over a long distance. As a configuration for increasing a length (hereinafter, referred to as an approaching distance) of approached portions between thin wire electrodes by arranging such thin wire electrodes in parallel with and approached each other, for example, a comb structure or a double spirally-wound structure may be employed. In addition, a structure itself for increasing an approaching distance between two wirings inside a predetermined plane area as possibly as can be is well known in the field of a semiconductor device and the like, and thus, such a structure may be employed as is necessary. In the present invention, "juxtaposing thin wire electrodes on a substrate" is not for specifying mutual directions of a plurality of thin wire electrodes placed on the substrate but represents that the thin wire electrodes on a same plane of the substrate with being separate from each other.

In a case where the first thin wire electrode 22 is used as a cathode, examples of the material of the first thin wire electrode 22 include gold (Au), platinum (Pt), silver (Ag), titanium (Ti), an alloy thereof, and carbon (C) and an allotrope thereof. In a case where the second thin wire electrode 23 is used as an anode, examples of the material of the second thin wire electrode 23 include silver (Ag), copper (Cu), iron (Fe), zinc (Zn), nickel (Ni), cobalt (Co), aluminum (Al), tin (Sn), chromium (Cr), molybdenum (Mo), manganese (Mn), magnesium (Mg), and an alloy thereof. It is noted that, in a case where silver and an alloy thereof are used as the first thin wire electrode 22, a material other than silver and an alloy thereof is used as the material for the second thin wire electrode 23.

The output (galvanic current value) depends on the combination of metal materials for the first thin wire electrode 22 and the second thin wire electrode 23. For example, when the combinations of silver/iron and gold/silver are compared with each other, the galvanic current value to be obtained in the combination of silver/iron is larger than that in the combination of gold/silver because the combination of silver/iron has a corrosion rate per area larger than that in the combination of gold/silver. On the other hand, in the combination of gold/silver, the service life is longer because the combination of gold/silver has smaller consumption of the electrodes. In this regard, silver has an effect of preventing the generation of mold in the galvanic-type sensor 11, and therefore, it is preferable to use silver as the first thin wire electrode 22 or the second thin wire electrode 23. Further, in a case where the first electrode 24 is made of the same material as that of the first thin wire electrode 22, and the second electrode 25 is made of the same material as that of the second thin wire electrode 23, the production process of the galvanic-type sensor 11 is simplified, and therefore, this is preferable.

In the galvanic-type sensor 11, when a galvanic current flows repeatedly, the metal of the anode electrode which is the second thin wire electrode 23 is ionized, and accordingly, the anode electrode (second thin wire electrode) is gradually consumed.

In order to address this problem with the laying density of the thin wire electrodes maintained, for example, the thickness of the anode electrode may be increased, or the width of the anode electrode may be increased instead of the width of the cathode electrode may be decreased. In a case where the spacing between the thin wire electrodes is very short, the influence of a slight increase in the spacing between the thin wire electrodes according to the consumption of the anode electrode on a result of the measurement increases. In a case where such an influence matters, for example, by using a principle that the consumption of the metal of the anode electrode is in proportion to the time integral of a galvanic current, a countermeasure of performing compensation calculation for the result of the measurement as the whole measurement system may be established.

The humidity measurement means 12 is arranged to be adjacent to the resistance measurement device 11, and is configured to measure the relative humidity near a portion in which an electrical characteristic (a galvanic current in a case where the resistance measurement device 11 is a galvanic-type sensor) is measured in the resistance measurement device 11 (hereinafter, in the same context, also simply referred to as a "measurement unit"), that is, near the two or more thin wire electrodes included in the resistance measurement device 11. Examples of such a humidity measurement means include a resistance change type humidity sensor that measures the amount of moisture absorbed by a dry and wet material such as a polymer or a ceramic as an electrical resistance, a capacitance type humidity sensor in which a polymer film or the like is used as a dryness/wetness responsive material, and a method in which a temperature sensor is used for monitoring a temperature as a saturated water vapor environment and determining a converted humidity and a humidity conversion system is used.

In a case where the sample 16, the resistance measurement device 11, and the humidity measurement means 12 are placed in the same enclosed space environment to sense and measure VFA, a method can be preferably used in which a platinum electrode is placed near the first thin wire electrode 22 and the second thin wire electrode 23 of the resistance measurement device 11, the temperature is monitored from the resistance value of the platinum electrode, and the converted humidity is determined. In particular in a case where platinum is used as the first thin wire electrode 22 or the second thin wire electrode 23 of the resistance measurement device 11, the temperature measurement unit of the humidity measurement means 12 adopting the above method can be easily provided in the resistance measurement device 11, leading to an advantage of reduction in the size and the cost of the entire VFA sensing and measurement device of the present invention.

The data extraction means 13 has a function of extracting output data from the resistance measurement device 11 when the relative humidity measured by the humidity measurement means 12 satisfies a predetermined humidity condition and sending the output data to the data analysis means 14.

As the predetermined humidity condition, the relative humidity is preferably 80% or more and less than 100%, and more preferably 80% or more and 95% or less as described above. In other words, when the data extraction means 13 extracts output data from the resistance measurement device 11, the space near the electrodes of the resistance measurement device 11 preferably has a relative humidity measured by the humidity measurement means 12 of 80% or more and less than 100%, and more preferably 80% or more and 95% or less. If the relative humidity satisfies this humidity condition, VFA can be sensed with high sensitivity, and VFA can be measured with high accuracy.

The data analysis means 14 is configured to compare the output data of the resistance measurement device 11 (the measurement data of the galvanic current in a case where the resistance measurement device 11 is a galvanic-type sensor) sent via the data extraction means 13 with calibration curve data or the like obtained in advance, and to output the amount of VFA corresponding to the output data. Alternatively, the data analysis means 14 is configured to compare the output data with a predetermined threshold value, and to output presence or absence of VFA in the sample 16 used for obtaining the output data.

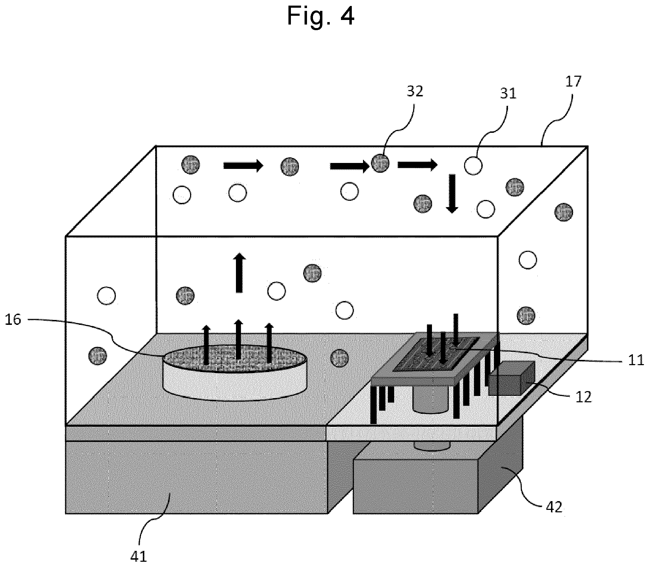

A specific aspect of the VFA sensing and measurement according to the present invention will be further described with reference to Fig. 4.

In the VFA sensing and measurement system shown in Fig. 4, a sample 16 containing VFA aqueous solution is placed on a base 41, together with a resistance measurement device 11, in an enclosed space 17 formed by an acrylic box or the like, and the VFA sensing and measurement system is configured so that water evaporated from the sample 16 (water vapor) 31 and VFA 32 can reach a measurement unit (a portion including at least electrodes) of the resistance measurement device 11. In the enclosed space 17, a humidity measurement means 12 is arranged to be adjacent to the resistance measurement device 11, and is configured to be capable of measuring the relative humidity near the electrodes of the resistance measurement device 11. Here, the system shown in Fig. 4 is preferably configured so that a temperature adjustment device 42 is arranged below the resistance measurement device 11 and thermally connected to the resistance measurement device 11, the temperature adjustment device 42 adjusts the temperature of the resistance measurement device 11, more accurately, the temperature of the measurement unit of the resistance measurement device 11, and thus the relative humidity of the space near the electrodes present in the measurement unit can be controlled. Examples of the temperature adjustment device 42 can include a Peltier device. In order to enhance the thermal conduction efficiency, the temperature adjustment device 42 and the resistance measurement device 11 are preferably connected thermally via a heat pump.

In the VFA sensing and measurement system configured as described above, the temperature adjustment device 42 adjusts the temperature while the humidity measurement means 12 monitors the relative humidity of the space near the electrodes of the resistance measurement device 11, and thus the relative humidity near the electrodes present in the measurement unit of the resistance measurement device 11 can be efficiently controlled so as to satisfy the humidity condition of immediately before occurrence of dew condensation between the electrodes, specifically, so that the relative humidity is 80% or more and less than 100%, or 80% or more and 95% or less.

The sample 16 and the measurement unit of the resistance measurement device 11 are placed in the enclosed space 17, and therefore when this system is used in an equilibrium state, the water 31 evaporated from the sample 16 and VFA 32 are co-adsorbed to the measurement unit of the resistance measurement device 11. At this time, the relative humidity of the space near the electrodes present in the measurement unit is controlled to a desired range (humidity condition of immediately before occurrence of dew condensation between the electrodes), so that VFA can be sensed and measured with high sensitivity and high accuracy.

Although a data extraction means 13 and a data analysis means 14 are not shown in Fig. 4, output data from the resistance measurement device 11 and the humidity measurement means 12 are to be sent to the data analysis means 14 via the data extraction means 13.

### Examples

### (Example 1)

In Example 1, VFA sensing and measurement system shown in Fig. 4 was produced. The results of examining characteristics of the system will be described.

It should be noted that the present invention is not limited to such a specific form, and the technical scope of the present invention is defined by the claims.

The resistance measurement device 11 was a galvanic-type sensor in which first thin wire electrodes 22 and second thin wire electrodes 23 were arranged in parallel with and approached each other into a comb shape on an insulating substrate 21 as shown in Fig. 2. The material of the first thin wire electrodes 22 was aluminum, and the material of the second thin wire electrodes 23 was gold. Each first thin wire electrode 22 and each second thin wire electrode 23 had a line width of 2 µm, a height of 200 nm, and a length of 1300 µm, and the spacing between the first thin wire electrode 22 and the second thin wire electrode 23 was 500 nm. The number of pairs including the first thin wire electrode 22 and the second thin wire electrode 23 was 92.

As the substrate 21, a substrate was used in which a thermal oxide film having a thickness of 100 nm is formed on a Si wafer. The substrate 21 had a flat part having a contact angle with water of 33°, and had a hydrophilic surface.

The humidity measurement means 12 was configured to monitor the temperature from the resistance value of the platinum electrode, and to determine the converted humidity from the temperature.

As the temperature adjustment device 42, a Peltier device was used.

As the sample 16, propionic acid aqueous solutions having a concentration set to 5 levels (0 mM, 10 mM, 20.7 mM, 100 mM, and 207 mM) were used. The amounts of the aqueous solutions were the same and were each 50 mL.

The enclosed space 17 was provided using a polypropylene resin box. The volume of the enclosed space 17 was 430 mL, and the shortest distance between a container containing the sample 16 and the resistance measurement device 11 was about 3 cm.

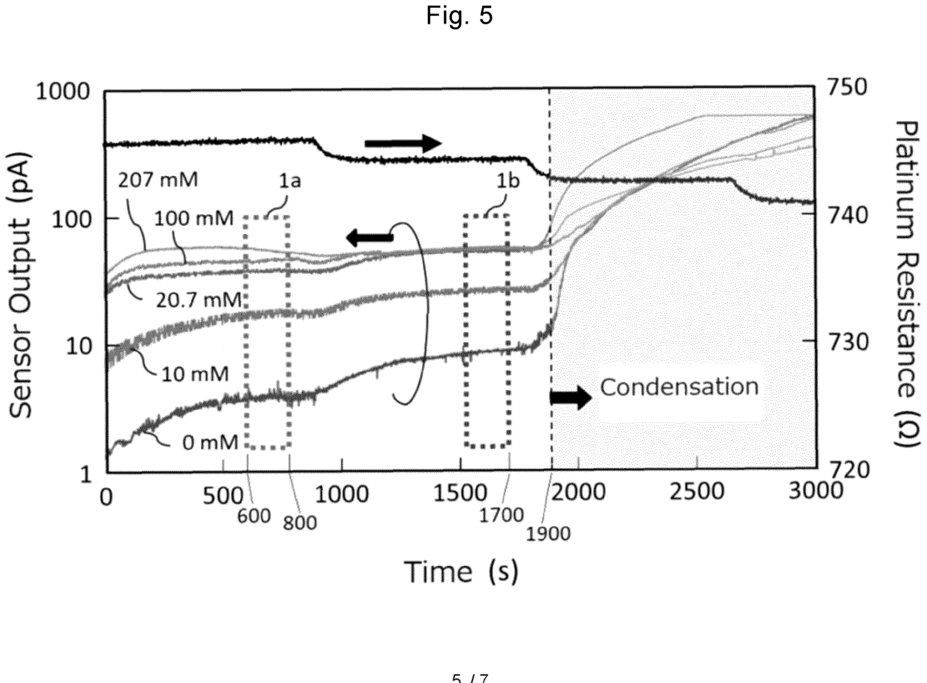

Fig. 5 shows the experimental results. When recording of output (sensor output) from the resistance measurement device (galvanic-type sensor) 11 was started, the time point was set to 0 (zero) s, and at the elapsed times of 900 s and 1800 s, the temperature adjustment device 42 changed (lowered) the temperature of the measurement unit of the sensor from the insulating substrate back surface to control the humidity of the space near the electrodes of the sensor. After the elapsed time of 1900 s, occurrence of dew condensation was visually confirmed (with a close-up camera). A time range of an elapsed time of 600-800 s was set as a zone 1a, and a time range of an elapsed time of 1500-1700 s was set as a zone 1b, as time ranges in which the sensor output had particularly high stability before the elapsed time of 1900 s. The relative humidity of the zone 1a was estimated to be 85% and the relative humidity of the zone 1b was estimated to be 92% from the resistance value (platinum resistance value) of the platinum electrode of the humidity measurement means, and thus the relative humidity was confirmed to be within the above-described preferable range of the humidity condition of immediately before occurrence of dew condensation between the electrodes of the resistance measurement device.

It can be seen that after the elapsed time of 1900 s at which dew condensation occurred, the magnitude relationship of the sensor output is not based on the concentration of propionic acid and that the behavior of the sensor output is based on the probability theory and irregular. Specifically, the sensor outputs of the samples having a propionic acid concentration of 0 mM and 10 mM are almost the same after the elapsed time of about 1950 s. After the elapsed time of 1900 s, the sensor output of the sample having a propionic acid concentration of 20.7 mM is higher than those of the samples having a propionic acid concentration of 100 mM and 207 mM, and after the elapsed time of about 2300 s, the sensor output of the sample having a propionic acid concentration of 100 mM is higher than that of the sample having a propionic acid concentration of 207 mM.

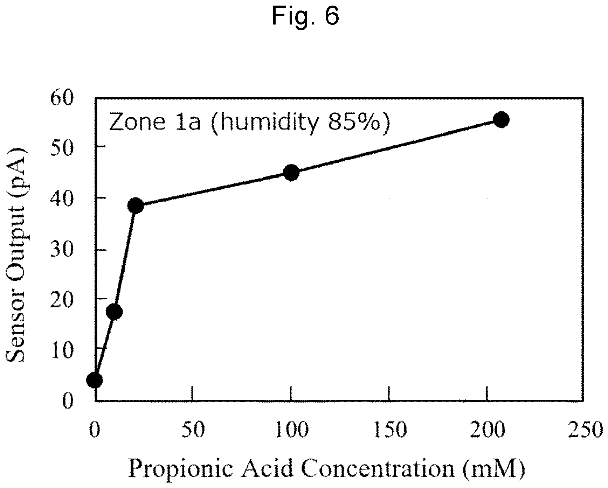

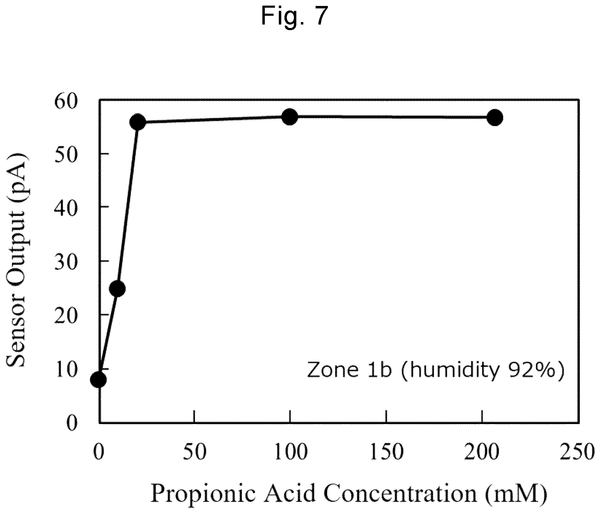

Meanwhile, in the zone 1a and the zone 1b immediately before dew condensation, the sensor output monotonically correlates with the propionic acid concentration. Fig. 6 shows the propionic acid concentration dependence of the sensor output in the zone 1a (at a relative humidity of 85%), and Fig. 7 shows that in zone 1b (at a relative humidity of 92%). In both Figs. 6 and 7, an inflection point is observed at a propionic acid concentration of about 20 mM. In the zone 1a, the characteristic curve is steep at a propionic acid concentration of 20 mM or less and relatively gentle at 20 mM or more, and in the zone 1b, the characteristic curve is steep at a propionic acid concentration of 20 mM or less and saturated at 20 mM or more. From these characteristic curves, it can be seen that propionic acid (VFA) having a concentration as low as 10 mM can be sensed and measured with sufficiently high linearity.

In the above example, the evaluation was performed with the concentration of the propionic acid aqueous solution. In addition, the Raoult's law can be used for conversion to determine the relative concentration and the partial pressure of propionic acid in a gas. Table 1 shows the conversion results. Here, the term "relative concentration" means the ratio to water (water vapor).

As shown in Table 1, it can be considered that the propionic acid contained in the sample in the form of an aqueous solution is present at a relative concentration and a partial pressure correlated with the concentration in the aqueous solution in the water vapor atmosphere in the enclosed space. Therefore, it can be said that the prototype system in the present example can achieve highly sensitive sensing and highly accurate measurement of VFA.

**[Table 1]**

| Propionic acid concentration in the sample solution (mM) | Partial pressure of propionic acid gas (Pa) @20°C | Relative concentration of propionic acid gas (%) |
|---|---|---|
| 0 | 0.000 | 0.000 |
| 10 | 0.070 | 0.018 |
| 20.7 | 0.145 | 0.037 |
| 100 | 0.702 | 0.180 |
| 207 | 1.453 | 0.373 |

### Industrial Applicability

According to the present invention, a method for sensing VFA with high sensitivity and measuring VFA with high accuracy and a device for such sensing and measurement are provided.

As described in the section of background art, VFAs are substances that greatly affect the productivity and quality improvement in dairy farming of cattle and the like, and also greatly relate to discharge of methane gas through digestion of food by cattle. Therefore, highly sensitive sensing and highly accurate measurement of VFA and feedback of the result are considered to improve the productivity and the quality in dairy farming greatly and contribute to prevention of global warming greatly.

Furthermore, the method for sensing VFA with high sensitivity and measuring VFA with high accuracy of the present invention leads to quantitative evaluation of activity of anaerobic bacteria, and can be expected to be widely used in various cases where VFA is generated by microbial decomposition, such as quality control in feed production, monitoring of a methane fermentation tank, operation management of a final disposal site (land fill), and environmental management of a paddy field or lake water.

### Reference Signs List

- 11: Resistance measurement device (galvanic-type sensor)
- 12: Humidity measurement means
- 13: Data extraction means
- 14: Data analysis means
- 15: Signal line
- 16: Sample
- 17: Enclosed space
- 21: Substrate (insulating substrate)
- 22: First thin wire electrode
- 23: Second thin wire electrode
- 24: First electrode (first collector electrode)
- 25: Second electrode (second collector electrode)
- 31: Water (water vapor)
- 32: VFA
- 41: Base
- 42: Temperature adjustment device (Peltier device)
- 101: VFA sensing and measurement device

## Claims

1. A method for sensing volatile fatty acid, the method comprising:
placing a sample in an enclosed space together with a resistance measurement device, wherein the sample is allowed to contain volatile fatty acid, the resistance measurement device measures an electrical characteristic caused by electrical resistance between at least two electrodes disposed to be adjacent to each other at a small spacing on an insulating substrate, and water vapor exists in the enclosed space;
measuring the electrical characteristic when a space near the at least two electrodes has a relative humidity satisfying a humidity condition of immediately before occurrence of dew condensation between the at least two electrodes; and
comparing an obtained measurement result with reference data obtained under a predetermined condition.

2. The method for sensing volatile fatty acid as claimed in claim 1, wherein the reference data is obtained by measuring the electrical characteristic using the sample when the space near the at least two electrodes has a relative humidity satisfying a humidity condition that does not cause dew condensation between the at least two electrodes, or obtained by measuring the electrical characteristic using a reference sample not containing volatile fatty acid or a reference sample containing volatile fatty acid at a certain rate when the space near the at least two electrodes has a relative humidity satisfying the humidity condition of immediately before occurrence of dew condensation between the at least two electrodes.

3. The method for sensing volatile fatty acid as claimed in claim 1 or 2, wherein the relative humidity is 80% or more and less than 100% under the humidity condition of immediately before occurrence of dew condensation between the at least two electrodes.

4. The method for sensing volatile fatty acid as claimed in claim 3, wherein the relative humidity is 80% or more and 95% or less under the humidity condition of immediately before occurrence of dew condensation between the at least two electrodes.

5. The method for sensing volatile fatty acid as claimed in any one of claims 1 to 4, wherein the at least two electrodes have a configuration in which a first thin wire electrode and a second thin wire electrode are alternately disposed in juxtaposition with each other in at least a partial region on the insulating substrate.

6. The method for sensing volatile fatty acid as claimed in claim 5, wherein the first thin wire electrode and the second thin wire electrode are alternately disposed at a specified spacing.

7. The method for sensing volatile fatty acid as claimed in claim 6, wherein the spacing is 100 nm or more and 1000 nm or less.

8. The method for sensing volatile fatty acid as claimed in any one of claims 5 to 7, wherein
the first thin wire electrode has an exposed surface including at least a part on which a first metal is formed, the second thin wire electrode has an exposed surface including at least a part on which a second metal is formed, the second metal being different from the first metal, and
a current flowing between the first thin wire electrode and the second thin wire electrode is measured.

9. The method for sensing volatile fatty acid as claimed in claim 8, wherein the first metal is selected from the group consisting of gold, platinum, silver, titanium, an alloy thereof, and carbon.

10. The method for sensing volatile fatty acid as claimed in claim 8 or 9, wherein the second metal is selected from the group consisting of silver, copper, iron, zinc, nickel, cobalt, aluminum, tin, chromium, molybdenum, manganese, magnesium, and an alloy thereof.

11. The method for sensing volatile fatty acid as claimed in any one of claims 1 to 10, wherein the insulating substrate has a hydrophilic surface.

12. The method for sensing volatile fatty acid as claimed in any one of claims 1 to 11, wherein the relative humidity is controlled by a temperature adjustment device thermally connected to the resistance measurement device.

13. The method for sensing volatile fatty acid as claimed in claim 12, wherein the temperature adjustment device is a Peltier device.

14. The method for sensing volatile fatty acid as claimed in any one of claims 1 to 13, wherein the sample is in a form of an aqueous solution.

15. A device for measuring volatile fatty acid, the device comprising a resistance measurement device, a humidity measurement means, a data extraction means, and a data analysis means, wherein
the resistance measurement device is configured to include at least two electrodes disposed to be adjacent to each other at a small spacing on an insulating substrate, to measure an electrical characteristic caused by electrical resistance between the at least two electrodes, and to output an obtained result,
the humidity measurement means is arranged to be adjacent to the resistance measurement device, and is configured to measure a relative humidity of a space near the at least two electrodes of the resistance measurement device, and to output an obtained result,
the data extraction means is configured to extract output data from the resistance measurement device when the relative humidity measured by the humidity measurement means satisfies a predetermined humidity condition, and
the data analysis means is configured to compare the output data extracted by the data extraction means with calibration curve data obtained in advance, and to output an obtained result.

16. The device for measuring volatile fatty acid as claimed in claim 15, wherein the at least two electrodes have a configuration in which a first thin wire electrode and a second thin wire electrode are alternately disposed in juxtaposition with each other in at least a partial region on the insulating substrate.

17. The device for measuring volatile fatty acid as claimed in claim 16, wherein the first thin wire electrode and the second thin wire electrode are alternately disposed at a specified spacing.

18. The device for measuring volatile fatty acid as claimed in claim 17, wherein the spacing is 100 nm or more and 1000 nm or less.

19. The device for measuring volatile fatty acid as claimed in any one of claims 16 to 18, wherein
the first thin wire electrode has an exposed surface including at least a part on which a first metal is formed, the second thin wire electrode has an exposed surface including at least a part on which a second metal is formed, the second metal being different from the first metal, and
the resistance measurement device is configured to measure a current flowing between the first thin wire electrode and the second thin wire electrode, and to output an obtained result.

20. The device for measuring volatile fatty acid as claimed in any one of claims 16 to 19, wherein the humidity measurement means includes humidity measurement electrodes that determine a humidity based on electrical resistance between the humidity measurement electrodes, and a material used for constituting the first thin wire electrode or the second thin wire electrode is used for constituting the humidity measurement electrodes.
